# FASCICULE DE BREVET EUROPEEN

(11) **EP 0 665 000 B1**
(45) Date de publication et mention de la délivrance du brevet: **02.10.1996**
(21) Numéro de dépôt: 94402894.3
(22) Date de dépôt: 15.12.1994
(51) Int. Cl.: A61K 7/00

(54) **Composition hydratante pour le traitement simultané des couches superficielles et profondes de la peau, son utilisation**
Hautbefeuchtendes Mittel zur gleichzeitigen Behandlung von oberen und tiefern Hautschichten sowie ihre Anwendung
Skinmoisturizing composition for simultaneous treatment of upper and inner skin layers, and its use

(30) Priorité: 30.12.1993 FR 9315864
(43) Date de publication de la demande: 02.08.1995
(73) Titulaire: L'OREAL, F-75008 Paris (FR)
(72) Inventeur: Ribier, Alain, F-75001 Paris (FR); Simonnet, Jean-Thierry, F-75011 Paris (FR); Nadaud, Jean-François, F-92140 Clamart (FR); Le Royer, Isabelle, F-78350 Jouy-en-Josas (FR)
(74) Mandataire: Lhoste, Catherine

(56) Documents cités:
- EP-A- 0 433 131
- EP-A- 0 559 502
- FR-A- 2 315 991
- FR-A- 2 408 387
- FR-A- 2 614 787
- INTERNATIONAL JOURNAL OF PHARMACEUTICS, vol. 62, ELSEVIER NL, page 75 V. GAGRIJELCIC ET AL. 'evaluation of liposomes as drug carriers into the skin by one-dimensional epr imaging'

## Description

L'invention se rapporte à une composition cosmétique et/ou dermatologique destinée à hydrater la peau, aussi bien du visage que du corps, voire même du cuir chevelu, et les ongles. Elle se rapporte plus particulièrement à une composition hydratante de ce type comprenant au moins un actif véhiculé par au moins deux catégories distinctes de vésicules lipidiques.

L'invention se rapporte aussi à une utilisation cosmétique de cette composition pour l'hydratation de la peau, et à un procédé d'hydratation de la peau.

On connaît de nombreux exemples de compositions cosmétiques ou dermatologiques destinées au traitement de la peau présentant un ou des actifs adaptés au traitement de la peau, encapsulés dans des vésicules ou sphérules lipidiques (appelées aussi liposomes).

On entend par vésicules ou sphérules lipidiques des particules formées d'une membrane constituée par un ou plusieurs feuillets concentriques, ces feuillets comportant une ou plusieurs couches bimoléculaires de lipides amphiphiles encapsulant une phase aqueuse. La phase aqueuse peut contenir des substances actives hydrosolubles et les couches bimoléculaires de lipides amphiphiles peuvent contenir des substances actives lipophiles.

Ces sphérules ont généralement un diamètre moyen compris entre 10 nm et 5000 nm.

Parmi les nombreux documents publiés concernant cette matière, on peut citer le certificat d'addition français 2408387 qui décrit une composition à base de dispersions aqueuses de sphérules lipidiques ioniques ou non ioniques encapsulant au moins une substance active. Plus précisément, ce document décrit des compositions contenant au moins deux dispersions de sphérules contenant des actifs différents, dans le but d'obtenir un système mixte, c'est-à-dire un système où l'on associe une première dispersion de sphérules contenant une première catégorie de substance active à une seconde dispersion de sphérules contenant une autre catégorie de substance active, qui permet aux deux catégories de substances d'agir simultanément au moment du traitement et d'obtenir éventuellement un effet synergique qui ne se produirait pas si l'on faisait agir successivement et séparément ces deux catégories de substances.

La demanderesse a maintenant mis au point des compositions cosmétiques et/ou dermatologiques hydratantes, permettant l'action simultanée de deux actifs différents et permettant, de plus, à ces actifs d'agir dans des zones différentes de la peau, c'est-à-dire dans les couches superficielles et dans les couches profondes de la peau, augmentant de ce fait très nettement l'efficacité de ces compositions et l'effet complémentaire ou synergique des actifs hydratants mis en oeuvre.

La demanderesse a également mis au point des compositions cosmétiques et/ou dermatologiques hydratantes, permettant au même actif d'agir simultanément dans les couches superficielles et dans les couches profondes de la peau, assurant un traitement plus complet et donc plus efficace de la peau.

Or, on sait combien il est important pour la peau d'être bien hydratée à la fois en superficie et dans les couches plus profondes de l'épiderme et du derme. L'exposition de la peau à l'atmosphère entraîne une déshydratation qui doit être compensée par un apport d'actifs hydratants.

Il est bien connu que la peau est constituée de couches superficielles, le *Stratum Corneum*, et de couches profondes, I'épiderme vivant et le derme. Or, on ne savait pas dans l'art antérieur délivrer spécifiquement tel actif dans les couches superficielles et, simultanément, le même ou tel autre actif dans les couches profondes.

La présente invention a pour objet une composition pour l'hydratation simultanée des couches superficielles et profondes de la peau, caractérisée en ce qu'elle comprend une première dispersion de vésicules lipidiques aptes à pénétrer dans les couches profondes de la peau et contenant au moins un actif choisi parmi les polyols, les sucres, les protides, les vitamines et leurs dérivés, les céramides, les acides gras essentiels,les stérols pour traiter ces couches profondes et une deuxième dispersion de vésicules lipidiques aptes à pénétrer dans les couches superficielles de la peau et contenant au moins un actif choisi parmi les polyols, les dérivés azotés d'acide carboxylique, les agents kératolytiques, les sels inorganiques, les lipoaminoacides pour traiter ces couches superficielles.

Selon un mode de réalisation particulier, les actifs contenus dans la première dispersion de vésicules et dans la deuxième sont les mêmes.

La demanderesse a utilisé un moyen de tri des vésicules permettant à l'homme de l'art de sélectionner aisément les vésicules lipidiques aptes à véhiculer l'actif dans les couches profondes de la peau, appelées vésicules de profondeur, et celles aptes à véhiculer l'actif dans les couches superficielles de la peau, appelées vésicules de surface.

Ce tri s'effectue sur la base de la constante de diffusion D d'une sonde introduite dans les vésicules. Cette sonde est l'ASL [l'iodure de N-(1-oxyl-2,2,6,6-tétraméthyl-4-pipéridinyl)-N-diméthyl-N-hydroxyéthylammonium] de formule :

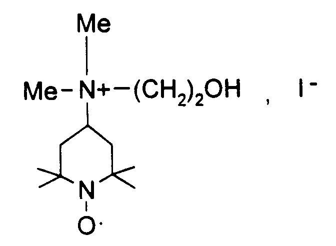

Les vésicules pour lesquelles la constante de diffusion D de la sonde dans le *Stratum Corneum* est > 1.10⁻⁷ cm² s⁻¹ sont les vésicules aptes à pénétrer dans les couches profondes de la peau.

Les vésicules pour lesquelles la constante de diffusion D de la sonde dans le *Stratum Corneum* est < 1.10⁻⁷ cm² s⁻¹ sont les vésicules aptes à véhiculer l'actif dans les couches superficielles de la peau.

Les vésicules de la première catégorie, dites de profondeur, sont en général à l'état fluide à température ambiante (autour de 20 °C), et celles de la seconde catégorie, dites de surface, sont en général à l'état gélifié à la température ambiante. Le moyen de reconnaître l'état des vésicules consiste à déterminer la température de transition de phase (lamellaire fluide-gel du lipide principal constituant leur membrane) par analyse thermique différentielle (ATD).

D'autres caractéristiques de ces vésicules sont en relation avec leur aptitude à délivrer l'actif plus ou moins en profondeur dans la peau. C'est en particulier le cas du taux d'encapsulation.

Le glucose est un marqueur classiquement utilisé pour ce type de détermination (cf. notamment Liposomes a practical approach de R.R.C. New, IRL Press (1990), p. 125-136).

Le taux d'encapsulation est exprimé par le volume de solution de glucose, encapsulée dans les vésicules, mesuré en µl par rapport à l'unité de poids (mg) des lipides constituant la membrane. Ce taux d'encapsulation est déterminé immédiatement après l'étape de séparation du glucose libre et du glucose encapsulé (T₀) ainsi que vingt-quatre heures après cette séparation (T₂₄ₕₑᵤᵣₑₛ).

La différence entre ces 2 déterminations successives illustre la perméabilité des vésicules vis-à-vis du glucose encapsulé, ce qu'on peut encore appeler potentiel d'encapsulation.

La première catégorie de vésicules (délivrant l'actif dans les couches profondes de la peau) présente un fort potentiel d'encapsulation des petites molécules hydrosolubles classiquement modélisées par le glucose, ce potentiel d'encapsulation se maintenant au moins 24 heures. La seconde catégorie de vésicules (délivrant l'actif dans les couches superficielles de la peau) ne retient pas le glucose à l'état encapsulé pendant le même temps.
Les lipides principaux constituant les vésicules de la première catégorie (actif délivré en profondeur) comportent au moins une chaîne grasse linéaire et saturée de longueur allant de 16 à 30 atomes de carbone, tels que les phospholipides hydrogénés (de plantes ou d'oeuf), les phospholipides synthétiques saturés tels que la dipalmitoyl-phosphatidylcholine, les alkyléthers ou alkylesters de polyols à une, deux ou trois chaînes grasses par molécule. Ces lipides sont utilisés seuls ou en mélange.

Les lipides principaux constituant les vésicules de la seconde catégorie (actif délivré en surface) sont choisis en particulier dans le groupe comprenant des lipides ioniques tels que notamment les phospholipides naturels à base de plantes ou d'oeuf, contenant des chaînes grasses insaturées ayant de 16 à 30 atomes de carbone ; des lipides non ioniques tels que notamment les alkyléthers ou les alkylesters de polyols à au moins une chaîne grasse par molécule, dont au moins une chaîne grasse de longueur inférieure à 16 atomes de carbone, tels que dans le lauryl polyglycéryl-6-cétéaryl glycoléther ; ainsi que leurs mélanges. Ce dernier est décrit en détail dans la demande de brevet FR 92-09603 déposée par L'Oréal.

On peut de façon connue incorporer dans la phase lipidique constituant la membrane lipidique des vésicules, au moins un additif choisi dans le groupe formé des stérols (phytostérols, cholestérol, phytostérols polyoxyéthylénés) ; des alcools, diols et triols à longue chaîne (phytanetriol), des amines à longue chaîne et leurs dérivés ammonium quaternaire ; des esters phosphoriques d'alcools gras et leurs sels alcalins (Na, K) tels que le dicétylphosphate, le dicétylphosphate de sodium, les alkylsulfates (cétylsulfate de sodium), les sels alcalins du cholestérol sulfate ou du cholestérol phosphate, le sel de sodium de l'acide phosphatidique, les lipoaminoacides et leurs sels tels que les acylglutamates de sodium.

On peut citer comme exemple de vésicules de la première catégorie (délivrant l'actif dans les couches profondes de la peau) les vésicules obtenues à partir des lipides suivants (nom CTFA) :
- A / cholestérol / lipoaminoacide caséique notamment dans un rapport pondéral 45/45/10 (où A est un cétyléther de triglycéryle commercialisé par la société Chimex sous le nom Chimexane NL) ;
- B / cholestérol / dicétylphosphate notamment dans un rapport pondéral 60/35/5 (où B est un mélange de mono-, di- et tri-cétyléther de triglycéryle commercialisé par la société Chimex sous le nom Chimexane NT) ;
- Span 40 (de chez Ici où Sorbitan palmitate) / cholestérol / acylglutamate de sodium commercialisé sous le nom de HS11 par la société Ajinomoto ,notamment dans un rapport pondéral 47,5/47,5/5 ;
- Stéarate de PEG 8 / cholestérol / acylglutamate de sodium avec notamment un rapport pondéral 47,5/47,5/5 (où le stéarate de PEG 8 est le polyéthylène glycol à 8 motifs d'oxyde d'éthylène commercialisé par Unichema sous le nom Stéarate de PEG 400) ;
- Stéarate de PEG 8 / cholestérol / phytanetriol / acylglutamate de sodium avec notamment un rapport pondéral 47,5/20/27,5/5 ;
- Lécithine hydrogénée / phytostérol polyoxyéthyléné à 5 motifs d'oxyde d'éthylène notamment dans un rapport pondéral 60/40 ;
- Distéarate de méthylglucose polyoxyéthyléné à 20 motifs d'oxyde d'éthylène / cholestérol / acylglutamate de sodium notamment dans un rapport pondéral 45/45/10 (le distéarate est par exemple commercialisé par Amerchol sous le nom Glucam E 20 distéarate par la société Amerchol) ;
- A / cholestérol / dicétylphosphate avec notamment un rapport pondéral 47,5/47,5/5 ;
- Distéarate de diglycéryle (par exemple Emalex DS G2 commercialisé par Nihon) / cholestérol / acylglutamate de sodium dans un rapport pondéral de 45/45/10 ;
- Mono- et di-stéarate de saccharose ( par exemple Grilloten PSE 141 G de chez Grillo) / cholestérol / acylglutamate de sodium notamment dans un rapport pondéral 45/45/10 ;
- Tristéarate de tétraglycéryle (par exemple Tetraglyn 3S de chez Nikkol) / cholestérol / acylglutamate de sodium notamment dans un rapport pondéral de 45/45/10.

On peut citer comme exemples de vésicules de la deuxième catégorie (délivrant l'actif dans les couches superficielles de la peau) les vésicules obtenues à partir des lipides suivants :
- Lécithine de tournesol ;
- Natipide II (lécithine de soja / éthanol / eau dans un rapport pondéral 60/20/20 commercialisé par Nattermann) ;
- C (lécithine de soja / cholestérol / propylène glycol dans un rapport pondéral 40/30/30 commercialisé par Nattermann sous le nom NAT 50 PG) ;
- D / dimyristylphosphate notamment dans un rapport pondéral 95/5 (où D est un éther de lauryl polyglycéryl-6-cétéarylglycol commercialisé par Chimex sous le nom Chimexane NS).

Le tableau I ci-après donne pour certaines vésicules obtenues à partir des lipides ci-dessus, la constante de diffusion D de l'ASL dans le *Stratum Corneum* et dans l'épiderme/derme ainsi que le taux d'encapsulation du glucose, et la température de transition de phase du lipide principal constituant la membrane. La constante de diffusion a été mesurée pour une concentration en ASL encapsulé de 0,35 % en poids par rapport au poids total de la composition.

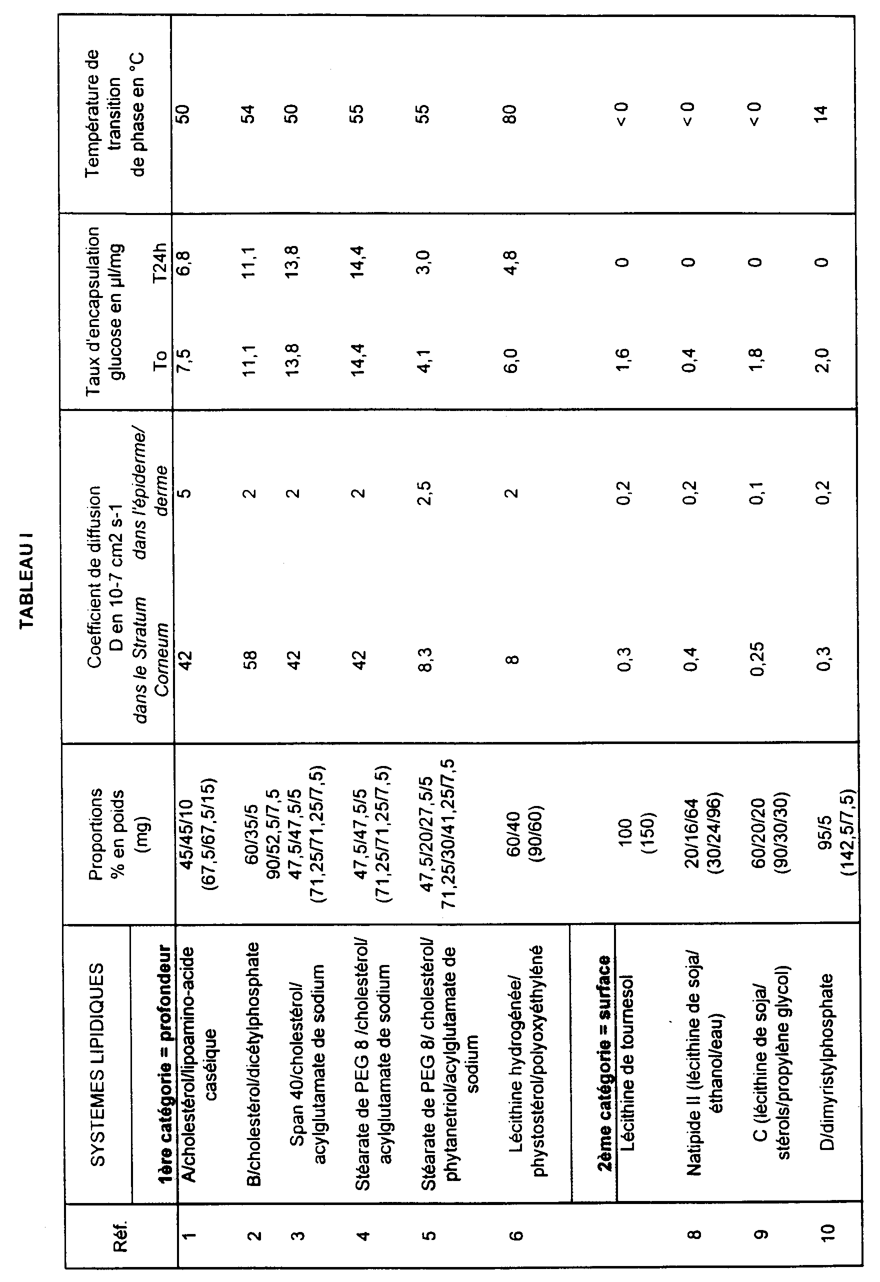

La mesure de la constante de diffusion D s'effectue par combinaison de deux méthodes utilisant une sonde paramagnétique, l'ASL : la résonance paramagnétique électronique (RPE) unidimensionnelle et périodique d'une part et l'imagerie cinétique RPE d'autre part. Ces deux méthodes sont décrites respectivement dans les articles International Journal of Pharmaceutics, 62 (1990) p. 75-79, Elsevier de V. Gabrijelcic et al. "Evaluation of liposomes as drug carriers into the skin by one-dimensional EPR imaging" et Periodicum Biologorum vol. 93, n° 2, p. 245-246, (1991) de V. Gabrijelcic et al. "Liposome entrapped molecules penetration into the skin measured by nitroxide reduction kinetic imaging".

La mesure du taux d'encapsulation s'effectue comme décrit dans le document RRC New cité précédemment et celle de la température de transition de phase comme décrit ci-dessus.

Avantageusement, on utilise simultanément plusieurs actifs dans chaque catégorie de vésicules, présentant la même fonction et/ou conférant à la peau, en surface et en profondeur, le même type d'effet ; les actifs de surface et de profondeur sont donc complémentaires.

Les actifs utilisables dans l'invention sont ceux classiquement utilisés dans le domaine cosmétique ou dermatologique.

Les actifs de profondeur sont choisis parmi les polyols, les sucres, les protides, les vitamines et leurs dérivés, les céramides, les acides gras essentiels et les stérols.

Comme polyols utilisables dans l'invention, on peut citer la glycérine, le sorbitol, le D-panthénol, et le mannitol.

Comme sucres utilisables dans l'invention, on peut citer le fructose, le galactose, le sucrose et la N-acétylglucosamine.

Comme protides utilisables dans l'invention, on peut citer les protéines végétales (blé, soja) et leurs hydrolysats, les peptides et polypeptides, l'hydroxyproline, la filagrine et ses précurseurs, le collagène et ses hydrolysats, l'élastine et ses dérivés, les acides aminés (alanine, glycine, citrulline, thréonine), les glycoaminoglycanes et leurs dérivés (sulfates).

Comme vitamines utilisables dans l'invention, on peut citer l'acide ascorbique (vitamine C) et ses dérivés (ascorbylphosphate de magnésium).

Comme acides gras essentiels utilisables dans l'invention, on peut citer l'acide linoléique et l'acide linolénique.

Comme stérols utilisables dans l'invention, on peut citer le cholestérol et ses dérivés (sulfate et phosphate), les phytostérols de soja.

Comme céramides utilisables dans l'invention, on peut citer les céramides et glycocéramides d'origine végétale ou animale, les céramides de synthèse, en particulier l'oleyldihydrosphingosine, les pseudocéramides.

Les actifs de surface sont choisis parmi les polyols, les dérivés azotés d'acide carboxylique, les agents kératolytiques et les sels inorganiques.

Comme polyols de surface utilisables dans l'invention, on peut citer la glycérine, le sorbitol, la diglycérine et les aminopolyols.

Comme dérivés azotés d'acide carboxylique utilisables dans l'invention, on peut citer l'urée, l'acide pyrrolidone carboxylique et ses dérivés (sel de sodium), l'acétamide et ses sels (méthylamine), les lipoaminoacides tels que la N-hexadécyloxycarbonyl-sérine ou la N-oléylglycylsérine.

Comme sels inorganiques utilisables dans l'invention, on peut citer les chlorures inorganiques, et en particulier le chlorure de sodium et le chlorure de magnésium.

Comme agents kératolytiques utilisables dans l'invention, on peut citer les alpha hydroxyacides, et en particulier les acides comme l'acide lactique, l'acide glycolique, l'acide malique, l'acide tartrique, l'acide citrique et leurs mélanges.

Les actifs peuvent représenter de 0,02 % à 10 % du poids total de la composition. De plus, les 2 catégories de vésicules peuvent contenir d'autres types d'actifs cosmétiques tels que les dépigmentants, les extraits végétaux.

Les compositions selon l'invention peuvent présenter toutes les formes galéniques normalement utilisées pour une application topique telles que les gels aqueux, les émulsions, les lotions, les pommades, les sérums et plus particulièrement les gouttelettes d'huile dispersées par les vésicules telles que décrites dans les brevets français FR-A-2485921 et FR-A-2490504.

De façon connue, dans les compositions de l'invention on peut trouver en plus des vésicules, une huile végétale, minérale, siliconée ou synthétique, dispersée dans une phase aqueuse et également des adjuvants hydrophiles comme les gélifiants, les conservateurs, les opacifiants, des adjuvants lipophiles tels que les parfums et les huiles essentielles, des pigments et des charges comme décrit dans les brevets français ci-dessus. L'huile dispersée peut représenter de 2 % à 40 % en poids par rapport au poids total de la composition et les adjuvants peuvent représenter, au total, de 0,1 % à 10 % en poids.

D'autres caractéristiques et avantages de l'invention ressortiront mieux de la description qui suit, donnée à titre illustratif et non limitatif.

### A) Obtention de vésicules lipidiques renfermant l'ASL

Les lipides constitutifs de la paroi des vésicules sont pesés et dissous dans 10 ml de méthanol. La solution alcoolique est alors transvasée dans un flacon rond de 50 ml à col rodé, que l'on place ensuite sur un évaporateur rotatif, de telle façon que le contenu soit thermostaté à la température de 30 °C. L'évaporation est poussée jusqu'au dépôt d'un film sec de lipides sur les parois du flacon.

3 ml d'une solution aqueuse 0,01 molaire d'ASL sont alors ajoutés dans le ballon qui est ensuite secoué à la main pendant environ 10 minutes, soit à la température ambiante (20 °C) pour les vésicules du tableau I référencées 7 à 10, soit à la température de 50 °C pour les vésicules référencées 1 à 6 du tableau I. On laisse alors le milieu s'équilibrer à température ambiante, pendant 2 heures, puis on place la dispersion dans un sac de dialyse et au contact de 500 ml d'eau distillée. La dialyse s'effectue pendant une nuit. Après une nuit, l'eau est changée et la dialyse est poursuivie pendant 4 heures supplémentaires.

Un fil de coton de 0,3 mm d'épaisseur est alors mis à tremper dans la dispersion de vésicules puis mis au contact d'une coupe de peau provenant d'une oreille de porc fraîchement récupérée dans un abattoir destiné à l'approvisionnement alimentaire.

L'échantillon d'oreille prélevé est rincé à l'eau et découpé en tranches de 1 mm d'épaisseur, 5 mm de large et 10 mm de long puis placé dans une cellule de maintien. Les mesures de diffusion de l'ASL dans la peau sont effectuées dans les 24 heures suivant le prélèvement de peau.

### B) Obtention de la composition cosmétique

### 1° Obtention des vésicules de première catégorie (diffusant en profondeur)

On prépare les vésicules (de profondeur) selon une méthode usuelle de co-fusion des constituants (voir tableau I) de la membrane choisis. Ainsi, on fond le constituant membranaire ayant le point de fusion T_{f} le moins élevé ; on ajoute les autres constituants membranaires puis on homogénéise sous agitation moyenne et enfin on hydrate partiellement en maintenant la température de fusion T_{f}, définie ci-dessus.

A la pâte obtenue, on ajoute une solution aqueuse d'au moins un premier actif, pour le traitement en profondeur. Une turbine est actionnée pendant 1 h 30 pour bien hydrater, en maintenant la température T_{f}. On rajoute au milieu réactionnel un ou plusieurs autres actifs pour le traitement en profondeur, on homogénéise et diminue la température du milieu jusqu'à la température ambiante (20 °C).

### 2° Obtention de vésicules de seconde catégorie (diffusant en surface)

On introduit à température ambiante (20 °C) et par simple agitation une solution aqueuse d'un (ou plusieurs) second actif pour le traitement en surface dans le mélange choisi de constituants devant former la membrane des vésicules de surface (voir tableau I). On obtient ainsi des vésicules de surface encapsulant le second actif de surface.

### 3° Obtention de la composition "double liposomes"

Au milieu contenant les vésicules de profondeur, on ajoute la phase grasse (huiles) de la composition et on la disperse (à température ambiante) sous agitation. On mélange alors le milieu réactionnel obtenu à celui contenant les vésicules de surface. Eventuellement, on ajoute alors les adjuvants tels que des conservateurs, un gélifiant que l'on peut neutraliser si nécessaire par une base (triéthanolamine ou soude) et des parfums, etc... .

Le produit obtenu se présente sous forme d'une crème blanche, douce et onctueuse utilisable dans le domaine cosmétique et/ou dermatologique pour hydrater la peau en surface et en profondeur. Cette crème peut être utilisée tous les jours.

On donne ci-après un exemple particulier de composition cosmétique conforme à l'invention.

### Exemple : Crème hydratante double liposomes

| -*Vésicules de profondeur :* | |
|---|---|
| Stéarate de PEG 8/cholestérol/acylglutamate de sodium dans un rapport pondéral 47,5/47,5/5 | 3,0 % |
| Glycocéramide de blé | 0,15 % |
| hydrolysat de collagène (actif) | 0,5 % |
| Hydrolysat de protéine de soja (actif) | 0,5 % |

| -*Vésicules de surface* | |
|---|---|
| Natipide II commercialisé par Natterman | 3,0 % |
| Glycérine (actif) | 0,5 % |
| Pyrrolidonecarboxylate de sodium (actif) | 0,4 % |

| -*Phase grasse:* | |
|---|---|
| Huiles végétales | 13,0 % |
| Beurre de karité | 4,0 % |
| Huile de silicone volatile | 4,0 % |
| Parfum | 0,4 % |

| -*Phase aqueuse*: | |
|---|---|
| Conservateur | 0,3 % |
| polymère carboxyvinylique (gélifiant) | 0,4 % |
| Soude | qsp pH 6 |
| Eau | qsp 100 % |

## Revendications

1. Composition hydratante pour le traitement simultané des couches superficielles et profondes de la peau, caractérisée en ce qu'elle comprend une première dispersion de vésicules lipidiques aptes à pénétrer dans les couches profondes de la peau et contenant au moins un actif choisi parmi les polyols, les sucres, les protides, les vitamines et leurs dérivés, les céramides, les acides gras essentiels et les stérols pour traiter ces couches profondes et une deuxième dispersion de vésicules lipidiques aptes à pénétrer dans les couches superficielles de la peau et contenant au moins un actif choisi parmi les polyols, les dérivés azotés d'acide carboxylique, les agents kératolytiques, les sels inorganiques, les lipoaminoacides pour traiter ces couches superficielles.

2. Composition selon la revendication 1, caractérisée en ce que les vésicules de la première dispersion assurent une diffusion d'ASL (iodure de N-(1-oxyl-2,2,6,6-tétraméthyl-4-pipéridinyl)-N-diméthyl-N-hydroxyéthyl-ammonium], dans le *Stratum Corneum,* > 1.10⁻⁷ cm² s⁻¹ et en ce que les vésicules de la seconde dispersion assurent une diffusion d'ASL dans le *Stratum Corneum*< 1.10⁻⁷ cm² s⁻¹.

3. Composition selon la revendication 1 ou 2, caractérisée en ce que les vésicules de la première dispersion sont à l'état fluide à température ambiante et les vésicules de la seconde dispersion sont à l'état gélifié à température ambiante.

4. Composition selon l'une quelconque des revendications 1 à 3, caractérisée en ce que les vésicules de la première dispersion assurent une encapsulation du glucose pendant au moins 24 heures et en ce que les vésicules de la seconde dispersion assurent une encapsulation du glucose pendant moins de 24 heures.

5. Composition selon l'une quelconque des revendications 1 à 4, caractérisée en ce que les vésicules de la première dispersion sont formées de lipides comportant au moins une chaîne grasse linéaire et saturée ayant de 16 à 30 atomes de carbone.

6. Composition selon l'une quelconque des revendications 1 à 5, caractérisée en ce que les vésicules de la première dispersion sont formées d'au moins un lipide choisi parmi les phospholipides naturels hydrogénés, les phospholipides synthétiques saturés, les alkyléthers de polyols à au moins une chaîne linéaire grasse, les alkylesters de polyols à au moins une chaîne grasse, et leurs mélanges.

7. Composition selon l'une quelconque des revendications 1 à 6, caractérisée en ce que les vésicules de la première dispersion sont formées d'au moins un lipide choisi parmi :
Cétyléther de triglycéryle / cholestérol / lipoaminoacide caséique ;
Mélange de mono-, di- et tri-cétyléther de triglycéryle / cholestérol / dicétylphosphate ;
Cétyléther de triglycéryle / cholestérol / dicétylphosphate ;
Sorbitan palmitate / cholestérol / acylglutamate de sodium ;
Stéarate de PEG 8 / cholestérol / acylglutamate de sodium ;
Distéarate de diglycéryle / cholestérol / acyglutamate de sodium ;
Mono- et di-stéarate de saccharose / cholestérol / acylglutamate de sodium ;
Stéarate de PEG 8 / cholestérol / phytanetriol / acylglutamate de sodium ;
Distéarate de méthylglucose polyoxyéthyléné à 20 moles d'oxyde d'éthylène / cholestérol / acylglutamate de sodium ;
Lécithine hydrogénée / phytostérol polyoxyéthyléné ;
Tristéarate de tétraglycéryle / cholestérol / acylglutamate de sodium.

8. Composition selon l'une quelconque des revendications 1 à 7, caractérisée en ce que les vésicules de la seconde dispersion sont formées de lipides choisis parmi les phospholipides ioniques naturels, contenant des chaînes grasses insaturées ayant de 16 à 30 atomes de carbone ; les alkyléthers ou les alkylesters de polyols à au moins une chaîne grasse par molécule, dont au moins une chaîne grasse de longueur inférieure à 16 atomes de carbone ainsi que leurs mélanges.

9. Composition selon l'une quelconque des revendications 1 à 8, caractérisée en ce que les vésicules de la seconde dispersion sont formées d'au moins un lipide choisi parmi :
Lécithine de tournesol ;
Lécithine de soja / éthanol / eau ;
Lécithine de soja / cholestérol / propylène glycol ;
Ether de lauryl polyglycéryl-6-cétéarylglycol / dimyristylphosphate.

10. Composition selon l'une quelconque des revendications 1 à 9, caractérisée en ce que l'actif de la première dispersion et celui de la seconde dispersion assurent la même fonction et/ou le même type d'effet.

11. Composition selon l'une quelconque des revendications 1 à 10, caractérisée en ce que les actifs de première et seconde dispersion sont les mêmes.

12. Composition selon l'une quelconque des revendications 1 à 11, caractérisée en ce que l'actif contenu dans la première dispersion est choisi parmi la glycérine, le sorbitol, le D-panthénol ; le mannitol, le fructose, le galactose ,le sucrose, la N-acétylglucosamine ; les protéïnes et leurs hydrolysats ; les peptides et polypeptides ; les acides aminés ; les glycoaminoglycanes et leurs dérivés ; l'acide ascorbique et ses dérivés ; les acides gras essentiels ; le cholestérol et ses dérivés, les céramides, glycoséramides et pseudocéramides.

13. Composition selon l'une quelconque des revendications 1 à 12, caractérisée en ce que l'actif contenu dans la seconde dispersion est choisi parmi la glycérine, le sorbitol, la diglycérine, les aminopolyols ; l'urée ; l'acide pyrrolidonecarboxylique et ses dérivés ; l'acétamide et ses sels ; les lipoaminoacides ; les chlorures inorganiques ; les alpha-hydroxyacides.

14. Composition selon l'une quelconque des revendications 1 à 13, caractérisée en ce qu'elle contient, en outre, une phase huileuse dispersée dans une phase aqueuse.

15. Composition selon l'une quelconque des revendications 1 à 14, caractérisée en ce qu'elle contient, en outre, des adjuvants hydrophiles et/ou lipophiles.

16. Utilisation cosmétique de la composition selon l'une quelconque des revendications 1 à 15 pour hydrater la peau.

17. Procédé cosmétique pour hydrater la peau, caractérisé en ce qu'il consiste à appliquer sur la peau une composition selon l'une quelconque des revendications 1 à 15.

## Claims

1. Moisturizing composition for the simultaneous treatment of the surface layers and deep layers of the skin, characterized in that it comprises a first dispersion of lipid vesicles which are capable of penetrating into the deep layers of the skin and which contain at least one active agent chosen from polyols, sugars, protides, vitamins and the derivatives thereof, ceramides, essential fatty acids and sterols, for treating these deep layers, and a second dispersion of lipid vesicles which are capable of penetrating into the surface layers of the skin and which contain at least one active agent chosen from polyols, nitrogen-containing carboxylic acid derivatives, keratolytic agents, inorganic salts and lipoamino acids, for treating these surface layers.

2. Composition according to Claim 1, characterized in that the vesicles of the first dispersion provide a diffusion of ASL [N-(1-oxyl-2,2,6,6-tetramethyl-4-piperidyl)-N,N-dimethyl-N-hydroxyethylammonium iodide] in the *stratum corneum* of > 1.10⁻⁷ cm² s⁻¹ and in that the vesicles of the second dispersion provide a diffusion of ASL in the stratum corneum of < 1.10⁻⁷ cm² s⁻¹.

3. Composition according to Claim 1 or 2, characterized in that the vesicles of the first dispersion are in the fluid state at room temperature and the vesicles of the second dispersion are in the gelled state at room temperature.

4. Composition according to any one of Claims 1 to 3, characterized in that the vesicles of the first dispersion provide an encapsulation of glucose for at least 24 hours, and in that the vesicles of the second dispersion provide an encapsulation of glucose for less than 24 hours.

5. Composition according to any one of Claims 1 to 4, characterized in that the vesicles of the first dispersion are formed of lipids composed of at least one linear and saturated fatty chain having from 16 to 30 carbon atoms.

6. Composition according to any one of Claims 1 to 5, characterized in that the vesicles of the first dispersion are formed of at least one lipid chosen from natural hydrogenated phospholipids, saturated synthetic phospholipids, polyol alkyl ethers containing at least one linear fatty chain, polyol alkyl esters containing at least one fatty chain, and mixtures thereof.

7. Composition according to any one of Claims 1 to 6, characterized in that the vesicles of the first dispersion are formed of at least one lipid chosen from:
Triglyceryl cetyl ether/cholesterol/casein lipoamino acid;
Mixture of triglyceryl mono-, di- and tricetyl ether/cholesterol/dicetyl phosphate;
Triglyceryl cetyl ether/cholesterol/dicetyl phosphate;
Sorbitan palmitate/cholesterol/sodium acylglutamate;
PEG 8 stearate/cholesterol/sodium acylglutamate;
Diglyceryl distearate/cholesterol/sodium acylglutamate;
Sucrose mono- and distearate/cholesterol/sodium acylglutamate;
PEG 8 stearate/cholesterol/phytanetriol/sodium acylglutamate;
Polyoxyethylenated methylglucose distearate containing 20 mol of ethylene oxide/cholesterol/sodium acylglutamate;
Hydrogenated lecithin/polyoxyethylenated phytosterol;
Tetraglyceryl tristearate/cholesterol/sodium acylglutamate.

8. Composition according to any one of Claims 1 to 7, characterized in that the vesicles of the second dispersion are formed of lipids chosen from natural ionic phospholipids containing unsaturated fatty chains having from 16 to 30 carbon atoms, polyol alkyl ethers or polyol alkyl esters containing one or more fatty chains per molecule, including at least one fatty chain with a length of less than 16 carbon atoms, and mixtures thereof.

9. Composition according to any one of Claims 1 to 8, characterized in that the vesicles of the second dispersion are formed of at least one lipid chosen from:
Sunflower lecithin;
Soya lecithin/ethanol/water;
Soya lecithin/cholesterol/propylene glycol;
Lauryl polyglyceryl-6-cetearyl glycol ether/dimyristyl phosphate.

10. Composition according to any one of Claims 1 to 9, characterized in that the active agent of the first dispersion and that of the second dispersion provide the same function and/or the same type of effect.

11. Composition according to any one of Claims 1 to 10, characterized in that the active agents of the first and second dispersions are the same.

12. Composition according to any one of Claims 1 to 11, characterized in that the active agent contained in the first dispersion is chosen from glycerine, sorbitol and D-panthenol; mannitol, fructose, galactose, sucrose and N-acetylglucosamine; proteins and the hydrolysates thereof; peptides and polypeptides; amino acids; glycosaminoglycans and the derivatives thereof; ascorbic acid and the derivatives thereof; essential fatty acids; cholesterol and the derivatives thereof, ceramides, glycoceramides and pseudoceramides.

13. Composition according to any one of Claims 1 to 12, characterized in that the active agent contained in the second dispersion is chosen from glycerine, sorbitol, diglycerol and aminopolyols; urea; pyrrolidonecarboxylic acid and the derivatives thereof; acetamide and the salts thereof; lipoamino acids; inorganic chlorides; alpha-hydroxy acids.

14. Composition according to any one of Claims 1 to 13, characterized in that it additionally contains an oily phase dispersed in an aqueous phase.

15. Composition according to any one of Claims 1 to 14, characterized in that it additionally contains hydrophilic or lipophilic adjuvants.

16. Cosmetic use of the composition according to any one of Claims 1 to 15 for moisturizing the skin.

17. Cosmetic process for moisturizing the skin, characterized in that it consists in applying to the skin a composition according to any one of Claims 1 to 15.

## Patentansprüche

1. Hydratisierende Zusammensetzung zur gleichzeitigen Behandlung der oberflächlichen und der unteren Hautschichten, dadurch gekennzeichnet, daß sie zur Behandlung der unteren Hautschichten eine erste Dispersion von Lipidvesikeln, die in die unteren Hautschichten eindringen können und mindestens einen unter den Polyolen, Zuckern, Proteidverbindungen, Vitaminen und deren Derivaten, Ceramiden, essentiellen Fettsäuren und Sterinen ausgewählten Wirkstoff enthalten, und zur Behandlung der oberflächlichen Hautschichten eine zweite Dispersion von Lipidvesikeln, die in die oberflächlichen Hautschichten eindringen können und mindestens einen unter den Polyolen, Stickstoffderivaten einer Carbonsäure, Keratolytika, anorganischen Salzen und Lipoaminosäuren ausgewählten Wirkstoff enthalten, enthält.

2. Zusammensetzung nach Anspruch 1, dadurch gekennzeichnet, daß die Vesikel der ersten Dispersion eine Diffusionskonstante der Diffusion von ASL (N-(1-Oxyl-2,2,6,6-tetramethylpiperidin-4-yl)-N-dimethyl-N-hydroxyethylammoniumiodid) in das *Stratum corneum* von > 1·10⁻⁷ cm² s⁻¹ und die Vesikel der zweiten Dispersion eine ASL-Diffusion in das *Stratum corneum* von < 1·10⁻⁷ cm² s⁻¹ gewährleisten.

3. Zusammensetzung nach Anspruch 1 oder 2, dadurch gekennzeichnet, daß die Vesikel der ersten Dispersion bei Raumtemperatur in einem flüssigen Zustand und die Vesikel der zweiten Dispersion bei Raumtemperatur im Gelzustand vorliegen.

4. Zusammensetzung nach einem der Ansprüche 1 bis 3, dadurch gekennzeichnet, daß die Vesikel der ersten Dispersion einen Einschluß von Glucose während mindestens 24 Stunden und die Vesikel der zweiten Dispersion einen Einschluß von Glucose während weniger als 24 Stunden gewährleisten.

5. Zusammensetzung nach einem der Ansprüche 1 bis 4, dadurch gekennzeichnet, daß die Vesikel der ersten Dispersion aus Lipiden gebildet sind, die mindestens eine lineare und gesättigte Fettkette mit 16 bis 30 Kohlenstoffatomen aufweisen.

6. Zusammensetzung nach einem der Ansprüche 1 bis 5, dadurch gekennzeichnet, daß die Vesikel der ersten Dispersion aus mindestens einem Lipid gebildet sind, das unter hydrierten natürlichen Phospholipiden, gesättigten synthetischen Phospholipiden, Alkylethern von Polyolen mit mindestens einer geradkettigen Fettkette und Alkylestern von Polyolen mit mindestens einer Fettkette sowie ihren Gemischen ausgewählt ist.

7. Zusammensetzung nach einem der Ansprüche 1 bis 6, dadurch gekennzeichnet, daß die Vesikel der ersten Dispersion aus mindestens einem Lipid gebildet sind, das ausgewählt ist unter:
Triglycerylcetylether / Cholesterin / Casein-Lipoaminosäure;
einem Gemisch aus Triglycerylmono-, -di- und - tricetylether / Cholesterin / Dicetylphosphat;
Triglycerylcetylether / Cholesterin / Dicetylphosphat;
Sorbitanpalmitat / Cholesterin / Natriumacylglutamat;
PEG-8-Stearat / Cholesterin / Natriumacylglutamat;
Diglyceryldistearat / Cholesterin / Natriumacylglutamat;
Saccharosemono- und -distearat / Cholesterin / Natriumacylglutamat;
PEG-8-Stearat / Cholesterin / Phytantriol / Natriumacylglutamat;
Methylglucosedistearat, das mit 20 mol Ethylenoxid polyethoxyliert ist / Cholesterin / Natriumacylglutamat;
hydriertes Lecithin / polyethoxyliertes Phytosterin;
Tetraglyceryltristearat / Cholesterin / Natriumacylglutamat.

8. Zusammensetzung nach einem der Ansprüche 1 bis 7, dadurch gekennzeichnet, daß die Vesikel der zweiten Dispersion aus Lipiden gebildet sind, die unter natürlichen ionischen Phospholipiden, die ungesättigte Fettketten mit 16 bis 30 Kohlenstoffatomen enthalten, Alkylethern oder Alkylestern von Polyolen mit mindestens einer Fettkette pro Molekül, wobei mindestens eine Fettkette weniger als 16 Kohlenstoffatome aufweist, und deren Gemischen ausgewählt ist.

9. Zusammensetzung nach einem der Ansprüche 1 bis 8, dadurch gekennzeichnet, daß die Vesikel der zweiten Dispersion aus mindestens einem Lipid gebildet sind, das ausgewählt ist unter:
Lecithin aus Sonnenblumen;
Lecithin aus Soja / Ethanol / Wasser;
Lecithin aus Soja / Cholesterin / Propylenglykol;
Polyglyceryl-6-cetearylglykollaurylether / Dimyristylphosphat.

10. Zusammensetzung nach einem der Ansprüche 1 bis 9, dadurch gekennzeichnet, daß der Wirkstoff der ersten Dispersion und der zweiten Dispersion die gleiche Wirksamkeit und/oder die gleiche Wirkungsart aufweisen.

11. Zusammensetzung nach einem der Ansprüche 1 bis 10, dadurch gekennzeichnet, daß die Wirkstoffe der ersten Dispersion und der zweiten Dispersion identisch sind.

12. Zusammensetzung nach einem der Ansprüche 1 bis 11, dadurch gekennzeichnet, daß der in der ersten Dispersion enthaltene Wirkstoff unter Glycerin, Sorbit, D-Panthenol, Mannit, Fructose, Galactose, Saccharose, N-Acetylglucosamin, Proteinen und ihren Hydrolysaten, Peptiden und Polypeptiden, Aminosäuren, Glykoaminoglykanen und ihren Derivaten, Ascorbinsäure und ihren Derivaten, essentiellen Fettsäuren, Cholesterin und seinen Derivaten, Ceramiden, Glykoceramiden und Pseudoceramiden ausgewählt ist.

13. Zusammensetzung nach einem der Ansprüche 1 bis 12, dadurch gekennzeichnet, daß der in der zweiten Dispersion enthaltene Wirkstoff unter Glycerin, Sorbit, Diglycerin, Aminopolyolen, Harnstoff, Pyrrolidoncarbonsäure und ihren Derivaten, Acetamid und seinen Salzen, Lipoaminosäuren, anorganischen Chloriden und α-Hydroxysäuren ausgewählt ist.

14. Zusammensetzung nach einem der Ansprüche 1 bis 13, dadurch gekennzeichnet, daß sie ferner eine in einer wäßrigen Phase dispergierte Ölphase enthält.

15. Zusammensetzung nach einem der Ansprüche 1 bis 14, dadurch gekennzeichnet, daß sie ferner hydrophile und/oder lipophile Hilfsstoffe enthält.

16. Kosmetische Verwendung der Zusammensetzung nach einem der Ansprüche 1 bis 15 zur Hydratisierung der Haut.

17. Kosmetisches Verfahren zur Hydratisierung der Haut, dadurch gekennzeichnet, daß es im Auftragen einer Zusammensetzung nach einem der Ansprüche 1 bis 15 auf die Haut besteht.
